# EUROPÄISCHE PATENTSCHRIFT

(11) **EP 1 272 201 B1**
(45) Veröffentlichungstag und Bekanntmachung des Hinweises auf die Patenterteilung: **24.11.2004**
(21) Anmeldenummer: 01929513.8
(22) Anmeldetag: 06.04.2001
(51) Int. Cl.: A61K 31/683, A61K 31/665, A61K 31/7032, A61K 31/7034, A61P 39/06

(54) **VERWENDUNG VON KOMPATIBLEN SOLUTEN ALS SUBSTANZEN MIT RADIKALFANGENDEN EIGENSCHAFTEN**
USE OF COMPATIBLE SOLUTES AS SUBSTANCES HAVING FREE RADICAL SCAVENGING PROPERTIES
UTILISATION DE SOLUTES COMPATIBLES EN TANT QUE SUBSTANCES AUX PROPRIETES DE PIEGEAGE DE RADICAUX

(30) Priorität: 12.04.2000 DE 10018225
(43) Veröffentlichungstag der Anmeldung: 08.01.2003
(73) Patentinhaber: Bitop Aktiengesellschaft Für Biotechnische Optimierung, 58453 Witten (DE)
(72) Erfinder: SCHWARZ, Thomas, 42799 Leichlingen (DE)
(74) Vertreter: Behrendt, Arne, Dipl.-Ing.
(86) Internationale Anmeldenummer: PCT/EP2001/003935
(87) Internationale Veröffentlichungsnummer: WO 2001/076572

(56) Entgegenhaltungen:
- EP-A- 0 354 474
- EP-A- 0 816 509
- EP-A- 0 965 268
- WO-A-00/07558
- WO-A-00/76528
- WO-A-01/58446
- WO-A-91/14435
- WO-A-97/38685
- WO-A-97/38686
- PT-A- 101 813
- US-A- 5 428 063
- DA COSTA ET AL: "An overview of the role and diversity of compatible solutes in bacteria and archaea" ADVANCES IN BIOCHEMICAL ENGINEERING, BIOTECHNOLOGY, SPRINGER, BERLIN, DE, Bd. 61, 1998, Seiten 117-153, XP002099622 ISSN: 0724-6145
- SAUER T ET AL: "BACTERIAL MILKING: A NOVEL BIOPROCESS FOR PRODUCTION OF COMPATIBLE SOLUTES" BIOTECHNOLOGY AND BIOENGINEERING, INTERSCIENCE PUBLISHERS, LONDON, GB, Bd. 57, Nr. 3, 1998, Seiten 306-313, XP000919323 ISSN: 0006-3592
- DATABASE WPI Week 198909 Derwent Publications Ltd., London, GB; AN 1989-065065 XP002181489 SENOO KEIKO ET AL.: "Antioxidant" & JP 01 016890 A (SHISEIDO CO LTD), 20. Januar 1989 (1989-01-20)
- LEHMACHER, A. ET AL: "Cyclic-2,3-diphosphoglycerate, protein stabilizer from thermophilic archaebacteria: synthesis and function" DECHEMA BIOTECHNOL. CONF. (1990), 4(PT. A, LECT. DECHEMA ANNU. MEET. BIOTECHNOL., 8TH, 1990), 415-18 , XP001035297
- MARTINS LIGIA O ET AL: "Accumulation of mannosylglycerate and Di-myo-inositol-phosphate by Pyrococcus furiosus in response to salinity and temperature." APPLIED AND ENVIRONMENTAL MICROBIOLOGY, Bd. 61, Nr. 9, 1995, Seiten 3299-3303, XP002114722 ISSN: 0099-2240

## Beschreibung

Die Erfindung bezieht sich auf radikalfangende bzw, antioxidativ wirkende Verbindungen und Kompositionen zur Behandlung von Krankheiten, zum Schutz der Haut und zum Schutz biologischer Moleküle und Strukturen sowie Nahrungsmittel, die als aktive Bestandteile verschiedene niedermolekulare Substanzen (kompatible Solute) aus extremophilen Mikroorganismen, namentlich Di-myo-inositolphosphat (DIP), cyclisches 2,3 diphosphoglycerat (cDPG), 1,1-Di-Glycerin-Phosphat (DGP), β-Mannosylglycerat (Firoin), β-Mannosylglyceramid (Firoin-A) oder/und Di-mannosyl-di-inositolphosphat (DMIP) und/oder ein Derivat, z.B. eine Säure, ein Salz oder Ester dieser Verbindungen als Substanzen mit radikalfangenden Eigenschaften enthalten.

Di-myo-inositolphosphat (DIP), cyclisches 2,3 diphosphoglycerat (cDPG), 1,1-Di-Glycerin-Phosphat (DGP), β-Mannosylglycerat (Firoin), β-Mannosylglyceramid (Firoin-A) oder/und Dimannosyl-di-inositolphosphat (DMIP) sind gemäß der Erfindung z.B. nützlich zum Schutz der Haut vor Umwelteinflüssen (z.B. in der Kosmetik oder Dermatologie) und in der Behandlung einer Krankheit, dem Schutz eines biologischen Materials und zur Abschwächung von Alterungsprozessen, welche durch Einwirkung freier Radikale und oxidativer Prozesse verursacht werden.

### STAND DER TECHNIK

### Radikalbildung

Freie Radikale sind Atome, Ionen oder Moleküle welche ein oder mehrere ungepaarte Elektronen in ihrer äußeren Elektronenhülle enthalten. Aufgrund dieser physikochemischen Charakteristik sind Radikale instabile, sehr reaktive und energiereiche Intermediate.

Reaktionen mit Sauerstoff sind die Basis für die Entstehung vieler freier Radikale. Aktiver Sauerstoff verursacht in vivo die Bildung von Superoxidradikalen, Wasserstoffperoxyd, Hydroxylradikalen und angeregter Singulett-Sauerstoff. Derartige Radikale bezeichnet man als Sauerstoffradikale oder auch reactiv oxygen species (Sauerstoffspezies, ROS). Sauerstoffradikale werden in vivo von Enzymen abgebaut oder durch radikalfangende natürliche Verbindungen, wie z.B. Ascorbinsäure, aufgefangen und umgesetzt. Superoxid wird durch Dismutase umgesetzt. Wasserstoffperoxyd wird von Katalase und Peroxidase entfernt. Singulett-Sauerstoff wird von Beta-Carotin und Tocopherol abgebaut.

Hydroxylradikale entstehen in vivo durch die Reaktion von Wasserstoffperoxid mit Superoxidradikalen. Hydroxylradikale sind sehr reaktiv und reagiert sehr schnell und unkontrolliert mit Zellbestandteilen. Die Existenzdauer eines einzelnen Hydroxylradikals ist dabei so kurz, dass Organismen keinen Mechanismus entwickelt haben, der sie in die Lage versetzt Hydroxylradikale zu entfernen.

Neben den hoch reaktiven Radikalen wie dem Hydroxylradikal werden moderat reaktive Radikale (Superoxidradikal) und persistente Radikale (Ascorbyl) unterschieden.

### Als typische Vertreter von Sauerstoffradikalen sind zu nennen:

Superoxid-Anion O⁻₂ Hydroxylradikal, Lipid-Peroxydradikal, radikalisches Sauerstoff, Wasserstoffperoxid sowie hypochlorige Säure.

Toxische Sauerstoffradikale entstehen als Nebenreaktion bei der Atmung.

In der Umwelt entstehen freie Radikale weiterhin durch Einwirkung ultravioletter Strahlung (besonders UV B), Zigarettenrauch, Ozon oder ionisierender Strahlung (z.B. während der Strahlungstherapie zur Behandlung von Krebserkrankungen, Röntgenstrahlung). Alkoholkonsum und Stress führen ebenfalls zu erhöhter Radikalbildung. Zusätzlich entstehen Radikale durch Reaktionen von Umweltgiften (z.B. Herbizide, Pestizide, Lösungsmittel), Schwermetallen oder Petrochemikalien. Selbst pharmazeutische Wirkstoffe und bestimmte Inhaltsstoffe von Nahrungsmitteln sind als Radikalbildner beschrieben.

### Natürliche Auswirkungen radikalischer Reaktionen

Es gibt viele klinische Berichte darüber, dass aktiver Sauerstoff und freie Radikale z.B. Membrangewebe eines lebendigen Körpers schädigen und hierdurch verschiedene Krankheiten verursacht werden.

Zellen werden oxidiert ("oxidativer Stress"), wenn freie Radikale auf sie einwirken. Hierbei werden in einer Kettenreaktion erneut Radikale gebildet, die ihrerseits wiederum zellschädigend wirken. Radikale werden auch gebildet, wenn Gewebe temporär von der Sauerstoffversorgung über das Blut abgeschnitten wird, wie dies z.B. bei Herzinfarkt, Schlaganfall oder der Organtransplantation der Fall ist. Bei der Lagerung biologischen Materials kommt es aufgrund von Radikalbildung auch bei niedrigen Temperaturen zu irreversiblen Schädigungen.

Radikale wirken zellschädigend und -zerstörend, indem sie deren natürliche Bestandteile, z.B. Zellproteine, Lipoproteine und Lipide verändern und zerstören. Durch radikalische Reaktionen werden zudem Nukleinsäuren geschädigt; es kommt zu Mutationen in der DNS. Radikale reagieren irreversibel mit freien Aminosäuren, Kohlenhydraten und gewebebildenden Makromolekülen (z.B. Collagen). Zellkompartimente wie z.B. Mitochondrien werden durch Radikale angegriffen. Radikale reagieren somit unkontrolliert mit Molekülen und makromolekularen Strukturen aller biochemischen Klassen.

Radikalische Reaktionen führen zu Alterungsprozessen biologischer Strukturen. Die radikalische Oxidation natürlicher Strukturen ist analog dem Rost bzw. der Korrosion von metallischen Werkstoffen. So entstehen z.B. Altersflecken oder trockene Haut durch Einwirkung von Radikalen. Die Alterung der Haut wird durch radikalisierende UV B und UV A Strahlung beschleunigt.

In Folge von Stress (z.B. aufgrund eines Jet lag) kommt es zu erhöhter Radikalbildung.

Freie Radikale sind in der Regel toxisch und verursachen sehr viele Krankheiten und Mangelerscheinungen. Bislang sind etwa 60 verschiedene Krankheiten beschrieben, die ihre Ursachen vollkommen oder teilweise in der Einwirkung freier Radikale haben. Zu diesen Krankheiten zählen AIDS, Allergien, Angina, Arthritis, Asthma, Artheriosklerose, Innere Blutungen, Zahnfleischblutung, Blutergüsse, Krebs, Katarakte, Durchblutungsprobleme, Zirrhose, Diabetes Typ 2, Austrocknen der Haut, Ödeme, Erschöpfungszustände, Heuschnupfen, Herzanfall, Hämorriden, erhöhter Blutdruck, Gewebsentzündung, Leber- und Nierenschäden, Impotenz, Gedächtnisverlust, Menstruationsbeschwerden, Migräne, Multiple Sklerose, Nachtblindheit, Parkinson, Venenentzündung, Prostataprobleme, Schuppenflechte, Respirationsprobleme, Netzhauterkrankungen, Senilität, Rheuma, Hautkrebs, Hautprobleme, Schlaganfall, Geschwollene Extremitäten, Krampfadern usw. Es konnte empirisch gezeigt werden, dass es einen Zusammenhang zwischen der oxidativen Schädigung durch Radikale und der Entstehung neurodegenerativer Krankheiten wie Huntington (HD), Parkinson (PD), Lou Gehrig (ALS) und Alzheimer (AD) gibt.

Jedoch sind nicht alle radikalischen Reaktionen toxisch. So wirken ubiquitäre Stickstoffoxidradikale (NO) in verschiedener Weise an der Regulation neurologischer, vaskulärer und immunologischer Funktionen mit. Gleichzeitig haben NO-Radikale jedoch auch schädlichen Einfluss. So reagiert NO mit Superoxidradikalen, wobei das noch reaktivere Peroxynitrit gebildet wird.

### Radikalfangende Substanzen / Antioxidantien

Es ist Stand der Technik, Radikalbildung durch Einsatz von sogenannten radikalfangenden Substanzen zu unterdrücken oder zu verhindern. Radikalfangende Substanzen reagieren mit den freien Radikalen, wobei Produkte gebildet werden, die nicht mehr radikalisch sind oder aber als Radikale zumindest weniger reaktiv sind, als die Ausgangsradikale. Radikalfänger haben somit eine Art "Blitzableiterfunktion" für freie Radikale. Wenn es sich bei der Radikalreaktion um eine Oxididationsreaktion handelt, spricht man bei Radikalfängern auch von Antioxidantien bzw. antioxidativ wirkenden Substanzen.

Natürliche Radikalfänger bzw. Antioxidantien sind Glutathion, Vitamin A oder dessen Ausgangssubstanz beta-Carotin, Vitamin C bzw. Analoga (Ascorbinsäure) und Vorstufen (Sorbit), Vitamin E, N-Acetyl-L-Cysteine. Für das Hormon Melatonin ist die radikalfangende Eigenschaft beschrieben.

Antioxidativa sind ebenfalls Pycnogenole und Proanthenole aus pflanzlichen Extrakten (Tee, Kiefernrinde). Weitere radikalfangende pflanzliche Extrakte enthalten Flavenoide als Radikalfänger. Proanthenole wirken z.B. Alterungsprozessen entgegen.

Zucker und Polyole sind bekannte Radikalfänger.

Kohlenwasserstoffverbindungen wie Pentadecanderivate, Fettsäurester von Baccatin, alphachlorierte Carbonate, 1,5-Anhydrofructose. Bestimmte essentielle Fettsäuren reduzieren ebenfalls den radikalbedingten schädigenden Einfluss.

Pyrrolpyrimidine bilden eine weitere Klasse antioxidativer Substanzen. Wichtiger Vertreter ist hier 5,5-Dimethyl-1-Pyrroline-N-Oxid (DMPO).

Nicorandil oder Sesamin werden als aktive Bestandteile von Radikalfängerkompositionen beschrieben.

Die kompatiblen Solute Glutamat Prolin, Trehalose, Mannitol, Sorbitol, Inositol und Betainderivate und -ester werden als mehr oder weniger gute Radikalfänger beschrieben.

Isoquercitrin, Toxerutin, Doxorubicin, Dihydrorobinetin, Hydrochinon- und Phenylenediaminderivate gelten ebenfalls als Radikalfänger.

Minerale wie Selen und Zink werden ebenfalls als Antioxidantien verwendet. Gleichzeitig wirkt Selen als Cofaktor zellulärer radikalneutralisierender Enzyme.

Enzyme, welche radikalische Reaktionen mit radikalfangenden Substanzen katalysieren, werden ebenfalls zur Verhinderung radikalischer Kettenreaktionen verwendet. Hier ist es Stand der Technik Dioxygenase, Monooxygenase, Oxidase, Hydroxylase, Superoxid-Dismutase, Gluthathione-Peroxidase und - Reduktase, Ekatalase, Katalase oder Thiol-spezifische antioxidative Enzyme.

Bestimmte Proteine wirken ebenfalls als körpereigene natürliche Radikalfänger. Hierzu zählen Transferrin, Lactoferrin, Ceruloplasmin, Albumin, Haptoglobin-Hemoplexin und Urat.

Wichtig bei der Anwendung dieser Radikalfänger ist, dass die Substanzen unter Radikaleinwirkung nicht selber toxische und reaktive Radikale bilden. Ideale Radikalfänger sind also solche, die nicht-toxisch, nicht-mutagen, nichtkarzinogen, nicht-antigen und nicht teratogen wirken.

### Quantifizierung des Radikalfangpotentials

Zur Quantifizierung radikalischer Reaktionen sind zahlreiche enzymatische und chemische Testverfahren beschrieben. Zur Standardisierung und zum Vergleich des radikalfangenden Potentials wurde der Radikalschutzfaktor (radical protection factor, RPF) zur Bestimmung der Radikalfangaktivität antioxidativer Produkte eingeführt. Das Prinzip der Methode zur Bestimmung des RPF-Werts basiert auf der Elektronen Spin Resonanz (ESR) und der wird in einem ESR-Spektrometer gemessen. Als Testsubstanz wird ein sehr reaktives Radikal benutzt, welches mit allen bekannten Antioxidantien reagiert.

### Applikationen von radikalfangenden Substanzen / Antioxidantien

Durch die Verwendung von radikalfangenden bzw. antioxidativ wirkenden Substanzen kann die Entstehung von radikalbedingten Zellveränderungen und Krankheiten reduziert oder sogar verhindert werden. Antioxidantien bzw. Radikalfänger wirken z.B. immunstimulierend, krebs- und entzündungshemmend.

In jedem Fall minimieren nicht-toxische Radikalfänger das Risiko an radikalinduzierten Krankheiten zu erkranken und erhöhen die Regenerationsfähigkeit von geschädigten Zellen und Geweben. Natürliche radikalinduzierte Prozesse wie z.B. die Alterung können verzögert werden.

Die Einsatzmöglichkeiten von Antioxidantien im Gesundheitsbereich, in der Kosmetik/Dermatologie und in der Ernährung sind daher sehr vielfältig. So ist es Stand der Technik, Vitamin A zum Hautschutz zu verwenden. Vitamin C schützt vor schädlichen Einflüssen des Tabakrauchs und schützt vor chronischer Bronchitis und anderen Lungenkrankheiten. Vitamin C unterstützt Heilungsprozesse und stimuliert die Zellregeneration. Durch Applikation von Vitamin E kann das Risiko von Herzinfarkt abgesenkt werden. N-Acetyl-L-Cystein mindert das Risiko der Krebsentstehung und die abnormale Gerinnung innerhalb von Blutgefäßen. Selen zerstört freie Radikale und schützt somit vor Krebs. Gluthation wirkt bei der Absenkung des Gehalts an Stoffwechselgiften und eliminiert freie Radikale. Gluthation mindert so das Entstehungsrisiko von Herzkrankheiten, Krebs, Immun- und Nervenkrankheiten. Gluthation wirkt zugleich als Regulator anderer wichtiger Antioxidantien.

Radikalfänger und Antioxidantien werden in Form von Tabletten, Kapseln, Granulaten oder Pulver oral verabreicht, sie werden als Trägerstoffe oder Excipients verwendet.

Entsprechend ist eine Aufgabe der vorliegenden Erfindung eine Substanzklasse bereitzustellen, die eine direkte Wirkung auf Hydroxylradikale und andere Radikale hat.

Gelöst wird die Aufgabe durch die Verwendung von kompatiblen Soluten ausgewählt aus der Gruppe bestehend aus Di-myo-inositolphosphat (DIP), cyclisches 2,3 diphosphoglycerat (cDPG), 1,1-Di-Glycerin-Phosphat (DGP) oder/und β-Mannosylglyceramid (Firoin-A) und/oder Derivaten dieser Verbindungen oder Kombinationen davon zur Herstellung von Mitteln zum Schutz von Organismen, Organen, Geweben, Zellen oder deren organischer Bausteine vor chemischen Radikalen und oxidativ wirkenden Verbindungen.

In überraschender Weise wurde gefunden, dass sich die kompatiblen Solute Di-myoinositolphosphat (DIP), cyclisches 2,3 diphosphoglycerat (cDPG), 1,1-Di-Glycerin-Phosphat (DGP) oder/und β-Mannosylglyceramid (Firoin-A) ein äußerst gutes Radikalfangpotential besitzen und somit nützliche Radikalfänger, Prophylaxe- und Heilmittel für Krankheiten, Kompositionen zum Schutz der Haut, als Schutzmittel für biologisches Material und Nahrungsmittelergänzungsstoffe sind.

Gegenstand der Erfindung ist daher die Verwendung mindestens einer Substanz ausgewählt aus der Gruppe folgender Verbindungen: Di-myoinositolphosphat (DIP), cyclisches 2,3 diphosphoglycerat (cDPG), 1,1-Di-Glycerin-Phosphat (DGP) oder/und β-Mannosylglyceramid (Firoin-A) oder ein Derivat, z.B. eine Säure, ein Salz oder Ester dieser Verbindungen zur Herstellung einer pharmazeutischen oder kosmetischen Zubereitung sowie Zubereitungen zum Schutze biologischer Moleküle und Strukturen sowie Nahrungsmittelzubereitungen.

Gemäß der Erfindung wird keine besondere Begrenzung auf die Dosisform der Komposition getroffen. Als Beispiele für die Dosisformen sind oral zu verabreichende Formulierungen, Einspritzungs-Zubereitungen, Zäpfchen-Zubereitungen, äußerlich anwendbare Zubereitungen (z.B. konditionierte Zugpflaster, Salben, Lotionen) oder Augenlösungen, zu nennen.

Krankheiten und Schädigungen von Organismen, die durch Radikale bzw. Oxidantien verursacht bzw. an deren Entstehung und Verlauf Radikale bzw. Oxidantien beteiligt sind, können durch die erfindungsgemäße Verwendung behandelt bzw. verhindert werden.

Die Dosis des aktiven Bestandteiles in jeder der Zusammensetzungen gemäß der vorliegenden Erfindung wird entsprechend dem Alter, Geschlecht und gesundheitlichen Zustand des Patienten bzw. Anwenders ausgewählt.

Die erfindungsgemäße Verwendung von kompatiblen Soluten schützt insbesondere organische Bausteine wie Proteine, Lipide, Fette oder Nukleinsäuren. Die Organismen sind insbesondere Mensch, Tiere und Mikroorganismen. Die Organe, die erfindungsgemäß mit den kompatiblen Soluten vor chemischen Radikalen und oxidativ wirkende Verbindungen geschützt werden können sind insbesondere Gewebe, Haut, Nieren, Herz, Extremitäten.

Vorzugsweise werden die kompatiblen Soluten in einer Konzentration von 0,001 M bis 2 M eingesetzt, indem diese in den zu schützenden Organismen, Organen, Geweben, Zellen oder deren organischer Bausteine in geeigneter Form zugeführt werden.

Erfindungsgemäß können die kompatiblen Solute auch zur Herstellung eines Arzneimittels zur Behandlung von Erkrankungen, die durch Einfluss von Radikalen und Oxidantien verursacht werden, eingesetzt werden. Es handelt sich dabei insbesondere um die folgenden Erkrankungen: des Herzens wie Herzinfarkt, Schlaganfall, Abstoßungsreaktionen nach Organtransplantation, AIDS, Allergien, Angina, Arthritis, Asthma, Arteriosklerose, Innere Blutungen, Zahnfleischblutung, Blutergüsse, Krebs, Katarakte, Durchblutungsprobleme, Zirrhose, Diabetes Typ 2, Ödeme, Erschöpfungszustände, Heuschnupfen, Herzanfall, Hämorriden, erhöhter Blutdruck, Gewebsentzündung, Leber- und Nierenschäden, Impotenz, Gedächtnisverlust, Menstruationsbeschwerden, Migräne, Multiple Sklerose, Nachtblindheit, Parkinson, Venenentzündung, Prostataprobleme, Schuppenflechte, Respirationsprobleme, Netzhauterkrankungen, Senilität, Rheuma, Hautkrebs, Schlaganfall, Geschwollene Extremitäten, Krampfadern sowie neurodegenerativer Krankheiten wie Huntington (HD), Parkinson (PD), Lou Gehrig (ALS) und Alzheimer (AD).

Desweiteren können die erfindungsgemäßen kompatiblen Solute zur Herstellung eines Kosmetikums und/oder dermatologischen Präparats zur Vorbeugung und Behandlung von Schädigungen und Veränderungen der Haut, die durch Einfluss von Radikalen und Oxidantien verursacht werden, verwendet werden.

Als Schädigungen und Veränderungen der Haut sind insbesondere Austrocknen der Haut, Hautprobleme, Dermatosen und Altersflecken zu nennen.

Die kompatiblen Solute lassen sich auch zur Herstellung eines Nahrungsmittels zur Vorbeugung und Behandlung von Schädigungen und Veränderungen des Organismus, die durch Einfluss von Radikalen und Oxidantien verursacht werden, verwenden.

Die Wirksamkeit der Zubereitung bestehend aus Lösungen der kompatiblen Solute Di-myo-inositolphosphat (DIP), cyclisches 2,3 diphosphoglycerat (cDPG), 1,1-Di-Glycerin-Phosphat (DGP) oder β-Mannosylglyceramid (Firoin-A) wurde durch folgendes Beispiel gezeigt:

Als Puffer wurde 100 mM Natriumphosphatpuffer, pH 6,8 verwendet. Jeweils eine Lösung der oben genannten Verbindungen wurde mit DPPH (2,2-Bis-(4-(1,1,3,3-Tetramethylbutyl)-Phenyl)1-Picrylhydrazyl; Endkonzentration: 0,125 mM) versetzt.

Es erfolgte die Bestimmung des RPF von Di-myo-inositolphosphat (DIP), cyclissches 2,3 diphosphoglycerat (cDPG), 1,1-Di-Glycerin-Phosphat (DPG) und β-Mannosylglyceramid (Firoin-A).

Der RPF, bestimmt nach der Methode von Herrling et al., Cosmetic World Journal (1998), gibt die Anzahl der Testradikale an, die von 1 mg der eingesetzten Substanz reduziert werden. Eingesetzt wurde das Testradikal DPPH.

### Ergebnisse

Zur genauen Bestimmung des RPF wurden jeweils 3 Messungen nach festgelegten Reaktionszeiten durchgeführt (30 min, 3 h und 24 h) und die Zeitkonstante der Gleichgewichtseinstellung bestimmt. In allen Fällen ist nach 24 h das Gleichgewicht nahezu vollständig erreicht, so dass die Messwerte zu diesem Zeitpunkt zur Bestimmung des RPF herangezogen werden. Als Referenzwert wurde der RPF (gegen DPPH) des Ascorbinsäure-2-phosphat - einem ausgezeichneten Radikalfänger - und Ectoin - einem sehr schwachen Radikalfänger - bestimmt. Die Ergebnisse sind in der folgenden Tabelle aufgeführt:

### Tabelle: RPF-Werte

Potential zum Radikalfang durch Di-myo-inositolphosphat (DIP), cyclisches 2,3 diphosphoglycerat (cDPG), 1,1-Di-Glycerin-Phosphat (DGP), und β-Mannosylglyceramid (Firoin-A).

| Probe | Konzentration mg/ml | Radikaltyp | Radikalkonz. M | Zeitkonstante H | RPF N/10¹⁴ |
|---|---|---|---|---|---|
| DIP | 5,1 | DPPH | 50,0 E-6 | 3,7 | 28 +/- 4 |
| Firoin-A | 12,0 | DPPH | 50,0 E-6 | 1,9 | 17 +/- 3 |
| DGP | 15 | DPPH | 50,0 E-6 | 2,4 | 24 +/- 3 |
| cDPG | 17,9 | DPPH | 50,0 E-6 | 2,9 | 9 +/- 0,3 |
| Ectoin | 60,3 | DPPH | 50,0 E-6 | | 0,04 |
| Ascorbinsäure-2-phosphat | 2,2 | DPPH | 50,0 E-6 | 1,7 | 38 +/-4 |

## Patentansprüche

1. Verwendung von kompatiblen Soluten ausgewählt aus der Gruppe bestehend aus Di-myo-inositolphosphat (DIP), cyclisches 2,3 diphosphoglycerat (cDPG), 1,1-Di-Glycerin-Phosphat (DGP) oder/und β-Mannosylglyceramid (Firoin-A) und/oder Derivaten dieser Verbindungen oder Kombinationen davon zur Herstellung eines Mittels zum Schutz von Organismen, Organen, Geweben, Zellen oder deren organischer Bausteine vor chemischen Radikalen und oxidativ wirkenden Verbindungen.

2. Verwendung nach Anspruch 1, wobei die organischen Bausteine Proteine, Lipide, Fette oder Nukleinsäuren sind.

3. Verwendung nach Anspruch 1 und/oder 2, wobei die Organismen Menschen, Tiere und Mikroorganismen sind.

4. Verwendung nach Anspruch 1 bis 3, wobei die Organe und Gewebe Haut, Nieren, Herz, Extremitäten sind.

5. Verwendung nach mindestens einem der Ansprüche 1 bis 4, wobei die kompatiblen Solute in einer Konzentration von 0,001 M bis 2 M eingesetzt werden.

6. Verwendung von kompatiblen Soluten zur Herstellung eines Arzneimittels zur Behandlung von Erkrankungen, die durch Einfluss von Radikalen und Oxidantien verursacht werden, wobei die kompatiblen Solute ausgewählt sind aus der Gruppe bestehend aus Di-myo-inositolphosphat (DIP), cyclisches 2,3 diphosphoglycerat (cDPG), 1,1-Di-Glycerin-Phosphat (DGP) o-der/und β-Mannosylglyceramid (Firoin-A) und/oder Derivaten dieser Verbindungen oder Kombinationen.

7. Verwendung nach Anspruch 6, wobei die Erkrankungen ausgewählt sind aus der Gruppe bestehend aus Erkrankungen des Herzens wie Herzinfarkt, Schlaganfall, Abstoßungsreaktionen nach Organtransplantation, AIDS, Allergien, Angina, Arthritis, Asthma, Arteriosklerose, Innere Blutungen, Zahnfleischblutung, Blutergüsse, Krebs, Katarakte, Durchblutungsprobleme, Zirrhose, Diabetes Typ 2, Ödeme, Erschöpfungszustände, Heuschnupfen, Herzanfall, Hämorriden, erhöhter Blutdruck, Gewebsentzündung, Leber- und Nierenschäden, Impotenz, Gedächtnisverlust, Menstruationsbeschwerden, Migräne, Multiple Sklerose, Nachtblindheit, Parkinson, Venenentzündung, Prostataprobleme, Schuppenflechte, Respirationsprobleme, Netzhauterkrankungen, Senilität, Rheuma, Hautkrebs, Schlaganfall, Geschwollene Extremitäten, Krampfadern sowie neurodegenerativer Krankheiten wie Huntington (HD), Parkinson (PD), Lou Gehrig (ALS) und Alzheimer (AD).

8. Verwendung von kompatiblen Soluten zur Herstellung eines Kosmetikums und/oder dermatologischen Präparats zur Vorbeugung und Behandlung von Schädigungen und Veränderungen der Haut, die durch Einfluss von Radikalen und Oxidantien verursacht werden, wobei die kompatiblen Solute ausgewählt sind aus der Gruppe bestehend aus Di-myo-Inositolphosphat (DIP), cyclisches 2,3 diphosphoglycerat (cDPG), 1,1-Di-Glycerin-Phosphat (DGP) oder/und β-Mannosylglyceramid (Firoin-A) und/oder Derivaten dieser Verbindungen oder Kombinationen.

9. Verwendung nach Anspruch 8, wobei Schädigungen und Veränderungen ausgewählt sind aus der Gruppe bestehend aus Austrocknen der Haut, Hautprobleme, Dermatosen und Altersflecken.

10. Verwendung von kompatiblen Soluten zur Herstellung eines Nahrungsmittels zur Vorbeugung und Behandlung von Schädigungen und Veränderungen des Organismus, die durch Einfluss von Radikalen und Oxidantien verursacht werden, wobei die kompatiblen Solute ausgewählt sind aus der Gruppe bestehend aus Di-myo-inositolphosphat (DIP), cyclisches 2,3 diphosphoglycerat (cDPG), 1,1-Di-Glycerin-Phosphat (DGP) oder/und β-Mannosylglyceramid (Firoin-A) und/oder Derivaten dieser Verbindungen oder Kombinationen.

## Claims

1. Use of compatible solutes selected from the group comprised of di-myo-inositol phosphate (DIP), cyclic 2,3 diphosphoglycerate (cDPG), 1,1-di-glycerol phosphate (DGP), or/and β-mannosylglyceramide (firoin-A) and/or derivates of these compounds or combinations thereof for the manufacture of an agent for protecting organisms, organs, tissues, cells or the organic buildings blocks thereof from chemical radicals and oxidatively active compounds.

2. Use according to claim 1, wherein the organic building blocks are proteins, lipids, fats or nucleic acids.

3. Use according to claim 1 and/or 2, wherein the organisms are human beings, animals and microorganisms.

4. Use according to claims 1 to 3, wherein the organs and tissues are skin, kidneys, heart, limbs.

5. Use according to at least one of claims 1 to 4, wherein the compatible solutes are used in concentrations of between 0.001 M and 2 M.

6. Use of compatible solutes for the production of a medicament for the treatment of diseases that are caused by free radical and oxidant activity, wherein the compatible solutes are selected from the group of di-myo-inositol phosphate (DIP), cyclic-2,3-diphosphoglycerate (cDPG), 1,1-di-glycerol phosphate (DGP) or/and β-mannosylglyceramide (firoin-A) and/or derivates of these compounds or combinations thereof.

7. Use according to claim 6, wherein the diseases are selected from the group comprised of heart diseases such as cardiac infarction, stroke, rejection after an organ transplant, AIDS, allergies, angina, arthritis, asthma, arteriosclerosis, internal bleeding, bleeding gums, hematomas, cancer, cataracts, circulation problems, cirrhosis, diabetes type II, edemas, fatigue, hay fever, heart attack, hemorrhoids, hypertension, inflamed tissues, liver and kidney damage, impotence, memory loss, menstrual disorders, migraine, multiple sclerosis, night blindness, Parkinson's disease, phlebitis, prostate problems, psoriasis, respiratory problems, retinopathy, senility, rheumatism, skin cancer, stroke, swollen limbs, varicose veins, as well as neurodegenerative diseases, such as Huntington's disease (or HD), Parkinson's disease (or PD), Lou Gehring's disease (or ALS) and Alzheimer's disease (or AD).

8. Use of compatible solutes for the production of a cosmetic and/or dermatological preparation for the prevention and treatment of skin damage or skin disorders caused by free radical and oxidant activity, wherein the compatible solutes are selected from the group of di-myo-inositol phosphate (DIP), cyclic-2,3-diphosphoglycerate (cDPG), 1,1-di-glycerol phosphate (DGP) or/and β-mannosylglyceramide (firoin-A) and/or derivates of these compounds or combinations thereof.

9. Use according to claim 8, wherein damage and disorders are selected from the group comprised of dehydration of the skin, skin problems, dermatoses, and age spots.

10. Use of compatible solutes for the production of a food product for the prevention and treatment of damage or alternation to organisms caused by free radical and oxidant activity, wherein the compatible solutes are selected from the group of di-myo-inositol phosphate (DIP), cyclic-2,3-diphosphoglycerate (cDPG), 1,1-di-glycerol phosphate (DGP) or/and β-mannosylglyceramide (firoin-A) and/or derivates of these compounds or combinations thereof.

## Revendications

1. Utilisation de solutés compatibles choisis parmi le groupe composé du di-myo-inositolphosphate (DIP), du 2,3-duphosphoglycérate cyclique (cDPG), du 1,1-di-glycérine-phosphate (DGP) et/ou du β-mannosylglycéramide (Firoïne A) et/ou de dérivés de ces composés ou de combinaison de ceux-ci pour fabriquer un agent de protection d'organismes, organes, tissus, cellules ou leurs constituants organiques contre des radicaux chimiques et des composés à effet oxydatif.

2. Utilisation selon la revendication 1, dans laquelle les constituants organiques sont des protéines, des lipides, des graisses ou des acides nucléiques.

3. Utilisation selon la revendication 1 et/ou 2 dans laquelle les organismes sont des êtres humains, des animaux et des micro-organismes.

4. Utilisation selon l'une des revendications 1 à 3, dans lequel les organes et tissus sont la peau, les reins, le coeur et les extrémités.

5. Utilisation selon au moins l'une des revendications 1 à 4, dans laquelle les solutés compatibles sont employés en une concentration de 0,001 M à 2 M.

6. Utilisation de solutés compatibles pour fabriquer un médicament pour le traitement de maladies qui sont provoquées par l'influence de radicaux et d'oxydants, les solutés compatibles étant choisis parmi le groupe composé du di-myo-inositolphosphate (DIP), du 2,3-diphosphoglycérate cyclique (cDPG), du 1,1-di-glycérine-phosphate (DGP) et/ou du β-mannosylglycéramide (Firoïne A) et/ou de dérivés de ces composés ou de combinaison de ceux-ci.

7. Utilisation selon la revendication 6, les maladies étant choisies parmi le groupe composé des maladies du coeur comme l'infarctus du myocarde, l'attaque d'apoplexie, les réactions de rejet avec une transplantation d'organe, le SIDA, les allergies, l'angine, l'arthrite, l'asthme, l'artériosclérose, les saignements internes, les saignements des gencives, les épanchements sanguins, le cancer, la cataracte, les problèmes d'irrigation sanguine, la cirrhose, le diabète de type 2, les oedèmes, les états d'épuisement, le rhume de foins, les attaques cardiaques, les hémorroïdes, la tension sanguine, les inflammations, les problèmes hépatiques et rénaux, l'impuissance, la perte de mémoire, les douleurs mensuelles, les migraines, la sclérose multiple, l'héméralopie, la maladie de Parkinson, les inflammations des veines, des problèmes de prostate, le psoriasis, les problèmes de respiration, les maladies rétiniennes, la sénilité, le rhume, le cancer de la peau, les attaques d'apoplexie, les extrémités enflées, les crampes et des maladies neuro-dégénératives comme les maladies de Huntington (HD), Parkinson (PD), lou Gehrig (ALS) et Alzheimer (AD).

8. Utilisation de solutés compatibles pour fabriquer un produit cosmétique et/ou une préparation dermatologique pour la prévention, le traitement, les dégradations et les modifications de la peau qui sont provoquées par l'influence de radicaux et d'oxydants, les solutés compatibles étant choisis parmi le groupe composé du di-myo-inositolphosphate (DIP), du 2,3-diphosphoglycérate cyclique (cDPG), du 1,1-di-glycérine-phosphate (DGP) et/ou du β-mannosylglycéramide (Firoïne A) et/ou de dérivés de ces composés ou de combinaison de ceux-ci.

9. Utilisation selon la revendication 8, les dégradations et modifications étant choisies parmi le groupe constitué des assèchements de la peau, des problèmes cutanés, des dermatoses et des taches de vieillissement.

10. Utilisation de solutés compatibles pour fabriquer un produit alimentaire pour la prévention, le traitement, les dégradations et les modifications de l'organisme qui sont provoquées par l'influence de radicaux et d'oxydants, les solutés compatibles étant choisis parmi le groupe composé du di-myo-inositolphosphate (DIP), du 2,3-diphosphoglycérate cyclique (cDPG), du 1,1-di-glycérine-phosphate (DGP) et/ou du β-mannosylglycéramide (Firoïne A) et/ou de dérivés de ces composés ou de combinaison de ceux-ci.
